# EUROPÄISCHE PATENTANMELDUNG

(11) **EP 1 201 140 A1**
(43) Veröffentlichungstag der Anmeldung: **02.05.2002**
(21) Anmeldenummer: 01122694.1
(22) Anmeldetag: 01.10.2001
(51) Int. Cl.: A23L 3/3508, A23K 3/03, A61L 2/16, C07C 59/01

(54) **Calciumdoppelsalze, deren Synthese und Verwendung als Konservierungsmittel**

(30) Priorität: 11.10.2000 DE 10050246
(71) Anmelder: Nutrinova Nutrition Specialties & Food Ingredients GmbH, 65929 Frankfurt am Main (DE)
(72) Erfinder: Raczek, Nico N., Dr., 65779 Kelkheim (DE); Mollenkopf, Christoph, Dr., 65929 Frankfurt am Main (DE)
(74) Vertreter: Schweitzer, Klaus, Dr. Dipl.-Chem.

(57) **Zusammenfassung**

Die vorliegende Erfindung betrifft Calciumdoppelsalze mit guten konservierenden Eigenschaften. Die genannten Salze können in Lebensmitteln, Futtermitteln, Haustierfutter, kosmetischen und pharmazeutischen Mitteln, Bedarfsgegenständen, Silagen, Biertrebern, Trester, Lebensmittelabfällen, Bierhefe, Destillationsschlempen und anderen Abfällen aus der Lebensmittelindustrie oder zur Lederbehandlung verwendet werden. Diese Stoffe weisen nach Zusatz bzw. Behandlung mit einem der Calciumdoppelsalze eine wesentlich verlängerte Haltbarkeit auf.

## Beschreibung

Die vorliegende Erfindung betrifft stabile Calciumdoppelsalze, welche in einem Molekül ein Calcium-Atom (Kation) und jeweils einen Rest zweier verschiedener organischer Säuren (Anion) im gleichen stöchiometrischen Verhältnis, vorzugsweise Säuren wie Ameisensäure, Essigsäure, Propionsäure, Sorbinsäure und Benzoesäure enthalten. Weiterhin betrifft die Erfindung Formulierungen, welche die Doppelsalze und einen Stabilisator und/oder ein Formulierungshilfsmittel enthalten.

Die Erfindung betrifft weiterhin ein Verfahren zur Herstellung der Doppelsalze, sowie die Verwendung der Salze zum Einsatz in Lebensmitteln, kosmetischen und pharmazeutischen Mitteln, Bedarfsgegenständen, Futtermitteln, wie zum Beispiel in Silagen, Biertrebern, Trester, Lebensmittelabfällen, Bierhefe, Destillationsschlempen und anderen Abfällen aus der Lebensmittelindustrie oder als konservierend wirkendes Mittel in technischen Produkten.

Physiologisch weniger bedenkliche, antimikrobiell wirkende Stoffe ersetzen zunehmend gesundheitlich oder aber für die Umwelt schädliche Substanzen, wie beispielsweise Antibiotika, formaldehydabspaltende Stoffe, halogenierte Substanzen, Borsäureabkömmlinge, u.v.a.m. in Lebensmitteln, Futtermitteln, Haustierfutter, kosmetischen und pharmazeutischen Mitteln, Bedarfsgegenständen, Silagen, Biertrebern, Trester, Lebensmittelabfällen, Bierhefe, Destillationsschlempen und anderen Abfällen aus der Lebensmittelindustrie oder bei der Lederbehandlung. Zu den physiologisch weniger bedenklichen Stoffen zählen die klassischen Konservierungsmittel, insbesondere Sorbinsäure, Propionsäure oder Benzoesäure. Diese organischen Säuren finden insbesondere in der Lebensmittelindustrie Anwendung (M. Jager u. E. Lück: Chemische Lebensmittelkonservierung, Berlin und Heidelberg 1995).

Vermehrt werden aber auch Mischungen organischer Säuren eingesetzt. Häufig nachteilig wirkt sich der flüssige Aggregatzustand, deren Flüchtigkeit oder eine schlechte Löslichkeit auch mancher ihrer Natrium-, Kalium-, Calcium- oder Magnesiumsalze aus. Oft ist eine schlechte Mischbarkeit oder eine Unverträglichkeit dieser Säuren festzustellen. So zeigen beispielsweise Mischungen von Ameisensäure und Benzoesäure eine schnelle Braunverfärbung, welche möglicherweise auf Oxidationsreaktionen zurückzuführen ist.

Konservierungsstoffsäuren wirken im allgemeinen in ihrer undissoziierten Form. Durch diese Abhängigkeit vom pH-Wert ergibt sich die Notwendigkeit, bei weniger sauren Lebensmitteln hohe Konzentrationen einiger Konservierungsstoffe einzusetzen die sich, beispielsweise bei der Anwendung von Propionsäure in Brot, sehr schnell sensorisch nachteilig bemerkbar machen können.

Unterschiedliche Produkteigenschaften der zu konservierenden Produkte, insbesondere unterschiedliche Konsistenz und unterschiedliche pH-Werte führen dazu, dass es keinen allgemein optimalen Konservierungsstoff gibt, sondern dass produktspezifische Lösungen bei der Konservierung erforderlich sind (M. Jager u. E. Lück: a. a. O.). Solche Lösungen bedeuten aber in vielen Fällen, dass neben einem Konservierungsstoff weitere Stoffe einzusetzen sind und damit die Notwendigkeit, z. B. verschiedene Konservierungsstoffe nebeneinander zu dosieren und vorrätig zu halten.

In der DE-A 197 39 319 werden säuregetränkte Carbonsäuresalze beschrieben, unter anderem auch Propionsäure getränktes Calciumformiat. Es handelt sich dabei nur um die Vermischung von Salzen und Säuren, wobei die Säure physikalisch das Salz adsorbiert, eine chemische Reaktion findet nicht statt.

Aufgabe der vorliegenden Erfindung war es ein definiertes Produkt herzustellen, welches die Vorteile zweier Konservierungsstoffsäuren verbindet. Insbesondere sollte ein lagerfähiges Produkt hergestellt werden, welches längere Transport- und Lagerzeiten bzw. schlechte Lagerbedingungen schadlos überstehen kann. Außerdem war es Aufgabe, Produkte herzustellen, welche leicht in der Lebensmittel- oder verwandten Industrien angewendet werden können.

Diese Aufgabe wird gelöst durch Calciumdoppelsalze mit zwei verschiedenen Säureeinheiten pro Doppelsalzmolekül. Es wurde gefunden, dass außer den seit langem bekannten Calciumsalzen der organischen Säuren eine ganze Reihe von Calciumdoppelsalzen mit zwei verschiedenen Säuren im Molekül hergestellt werden können, bei denen verschiedene Kombinationen der Säureanionen möglich sind.

Die Erfindung betrifft dementsprechend Calciumdoppelsalze der Formel I

Ca(R¹)(R²) (I)

worin R¹ und R² verschieden sind und jeweils OOC-R³ bedeuten, mit R³ = gesättigtes oder ein- oder mehrfach ungesättigtes C₁-C₅-Alkyl, C₁-C₅-Hydroxyalkyl oder Phenyl. Bevorzugt sind Verbindungen, in denen gängige Konservierungsstoffsäuren eingesetzt werden, also Verbindungen der Formel (I), in denen R³ Methyl, Ethyl, Hydroxyethyl (= Lactatanion), Propyl, 1,3-Hexadienyl (=Sorbatanion) oder Phenyl bedeutet. Ganz besonders bevorzugt bedeutet R¹ Propionat und R² Sorbat.

Wird hingegen versucht, Natrium- oder Kaliumsalze herzustellen, können je nach molarem Verhältnis der eingesetzten Edukte (beispielsweise bei der Umsetzung von Natriumacetat mit Milchsäure oder Kaliumsorbat mit Propionsäure oder Natriumbenzoat mit Essigsäure) nur unzureichend stabile und definierte Produkte erhalten werden. Diese Produkte verflüssigen und verfärben sich ohne ausreichend große Träger/Stabilisatormengen bereits nach kurzer Zeit dunkelbraun, so daß hier kein praktisch nutzbares Produkt gewonnen werden kann. Dies trifft auch auf die in DE 197 39 319 beschriebenen getränkten Salze zu, die ohne entsprechende Zusätze verklumpen und bei Raumtemperatur nach kurzer Lagerdauer eine Braunverfärbung aufweisen. Überraschenderweise zeigen die erfindungsgemäßen Calciumdoppelsalze dieses Verhalten nicht.

Eine Möglichkeit zur Herstellung dieser Doppelsalze besteht in der Reaktion von einem Alikalisalz, bevorzugt einem Natrium- oder Kaliumsalz der einen Säure mit dem Calciumsalz der anderen Säure. Vorzugsweise werden Calciumsalze der Ameisensäure, Essigsäure, Propionsäure, Sorbinsäure und Benzoesäure verwendet. Die so entstandenen definierten Calciumdoppelsalze zeigen eine ausgezeichnete Stabilität. Weiterhin besteht die Möglichkeit einer Reaktion durch den Umsatz zweier Säuren in stöchiometrischen Verhältnissen mit Calciumhydroxidlösung. Durch beide Methoden entstehen nach Isolierung und Trocknung, definierte weiße, amorphe oder feinstkristalline Pulver, welche in einem Molekül ein Calcium-Atom und jeweils einen (deprotonierten) Rest zweier verschiedener organischer Säuren in gleichen stöchiometrischen Verhältnis enthalten. Diese Doppelsalze sind im Gegensatz zu getränkten Salzen und bei Verwendung von Alkalisalzen der gleichen Säure erhaltenen Produkten sehr stabil und zeigen auch nach längerer Lagerung keine Braunverfärbungen.

Bei der Herstellung wird vorzugweise in wäßrigen, gesättigten Lösungen bei Temperaturen zwischen 15 und 40 °C, bevorzug zwischen 20 und 25 °C gearbeitet. Das molare Verhältnis der zu mischenden Salze beträgt 1 : 0,2 bis 1 :1, bevorzugt 1 : 0,35 bis 1 : 0,5. Die Zeit der Vermischung und der Niederschlagsabtrennung dauert insgesamt zwischen 1 und 15 min., vorzugsweise zwischen 2 und 10 min. Die Produkte können zwischen 25 und 100 °C, vorzugsweise zwischen 30 und 60 °C und bei 30 bis 500 hPa, bevorzugt zwischen 80 und 150 hPa getrocknet werden. Als Nebenprodukt können in geringeren Anteilen Calciumsalze der einzelnen Säuren entstehen.

Die definierten Doppelsalze von vorzugsweise Ameisensäure, Essigsäure, Propionsäure, Sorbinsäure, Benzoesäure, zeigen eine ausgezeichnete antimikrobiologische Wirksamkeit. Besonders gut werden Mikroorganismen durch die Doppelsalze aus zwei ausgesprochenen Konservierungsstoffsäuren gehemmt, beispielsweise durch das Calciumdoppelsalz aus Sorbin- und Propionsäure.

Die erfindungsgemäßen Doppelsalze lassen sich auf den Gebieten verwenden, auf denen herkömmliche Konservierungsstoffe eingesetzt werden. Über die konservierende Wirkung hinaus wird mit den erfindungsgemäßen Salzen bei gleichzeitiger Verwendung calciumempfindlicher Verdickungs- und Geliermittel eine Verfestigung erreicht, für die in anderen Fällen Calciumsalze gezielt zugesetzt werden müssen. Zu den Einsatzgebieten gehören:

### Getränke

Die erfindungsgemäßen Doppelsalze können zur Haltbarmachung von alkoholfreien und alkoholhaltigen Getränken sowie Konzentraten zur Getränkeherstellung eingesetzt werden. Sie verhindern insbesondere unerwünschte Gärungen durch Hefen. Einsatzkonzentrationen liegen zwischen 0,05 - 5 g/kg, vorzugsweise bei 0,1 bis 1 g/kg.

### Obsterzeugnisse, getrocknetes Obst, Konfitüren und Marmeladen

Eine sinnvolle Einsatzkonzentration liegt bei etwa 0,1 bis 5 g/kg, vorzugsweise 0,25 bis 2 g/kg. Insbesondere bei zuckerreduzierten und verdickungsmittelhaltigen Erzeugnissen wird nicht nur ein mikrobiologische Stabilisierung erreicht, sondern kann auch das für das Gelieren von niederveresterten Pektinen erforderliche Calcium geliefert werden.

Ein Zusatz der Doppelsalze sollte zweckmäßigerweise bei erhitzten Produkten gegen Ende des Erhitzungsprozesses erfolgen, insbesondere, wenn dabei Verluste an flüchtigen Säuren möglich sind.

### Sauergemüse, Feinkostsalate und Würzsoßen

Bei Gemüsesauerkonserven wie Essiggurken, mixed pickles und ähnlichen und milchsauer vergorenen Gemüsen wie Sauerkraut und Oliven werden die erfindungsgemäßen Salze den Aufgussflüssigkeiten in Mengen von bis zu 0,1 bis 5 g/kg, vorzugsweise 0,25 bis 3 g/kg. Im gleichen Konzentrationsbereich eignen sie sich auch zur Verwendung in Feinkostsalaten, Würzsoßen, und verwandten Produkten wie Senf.

### Backwaren und Teige

Die erfindungsgemäßen Doppelsalze lassen sich in Backwaren der verschiedensten Art, die verderbsanfällig sind, Backwarenfüllungen, teil- und vorgebackenen Erzeugnissen sowie Fertigteigen problemlos einsetzen. Dafür sind je nach Produkt und gewünschter Verlängerung der Haltbarkeit Mengen bis zu 0,5 bis 5 g/kg sinnvoll.

Zum Einsatz in feinen Backwaren, insbesondere in Backwaren mit ungebackenen Füllungen basierend auf Alginaten, können thermoreversible Gele erhalten werden. Ebenso können ähnliche Effekte beim Einsatz von Stärke oder anderen Verdickungsmitteln erreicht werden. Füllungen jeder Art in und auf Backwaren, Baisers, Schokoladensoßen, Salatsoßen und Eiscreme können nicht nur in ihrer mikrobiologischen Haltbarkeit wesentlich verlängert werden, sondern weitere stabilisierende oder destabilisierende Effekte können beim Einsatz der Calciumdoppelsalze erreicht werden. Dazu ist ein Zusatz von 0,5 bis 5 g/kg zu den entsprechenden Produkten notwendig. Die Zusatzmenge hängt dabei wesentlich von der Konzentration der Alginate, Stärke bzw. anderen Verdickern ab. Das erfindungsgemäße Salz kann zu einer trockenen Vormischung gegeben werden, die zu einem späteren Zeitpunkt - i.a. durch Zugabe von Milch, Sahne oder Wasser - fertig zubereitet wird oder bei bestimmten Zubereitungen beispielsweise auch in Fett verschäumt oder im Vollei homogenisiert werden, wobei dadurch die Aufschlagfestigkeit bzw. eine Stabilität der Masse erhöht wird.

### Käse

Ein Zusatz von 0,5 bis 2 g/kg bezogen auf den gesamten Käselaib, der erfindungsgemäßen Salze reicht i. d. R. zur Konservierung von gereiften Käsen aus. Ein Zusatz der erfindungsgemäßen Doppelsalze zum Salzbad hält die so behandelte Käse für mehrere Wochen schimmelfrei. Vorzugsweise werden den Salzbädern von Hartkäsen täglich 0,5 - 10 g/l der Salze, in Abhängigkeit von der Löslichkeit, zugesetzt. Eine Oberflächenbehandlung mit wäßriger Lösung durch Tauchen oder Besprühen mit wäßrigen Lösungen oder Suspensionen der Salze in einer Konzentration von 10 g/l und Nachbehandlung in Intervallen von 1 bis 5 Wochen hält reifende Käse frei von Schimmel. Bei Käsen, die eine Sprühbehandlung nicht zulassen, ist auch das Auftragen einer viskos eingestellten Suspension oder das Aufbringen der trockenen Salze möglich.

Bei Schmelzkäse können diese Salze zusammen mit den Schmelzsalzen zugesetzt werden.

Desgleichen erreicht man bei Speisequark und Sauermilcherzeugnissen durch Zusatz von bis zu 0,5 bis 2 g/kg der Salze eine gute Haltbarkeitsverlängerung.

### Fettemulsionen

Zum Einsatz in Fettemulsionen wie Margarine, Mayonnaise, Salatsoßen und Dressings werden vorzugsweise besser lösliche Doppelsalze verwendet. Die üblichen Einsatzmengen liegen bei bis zu 0,25 bis 3 g/kg. Der Zusatz erfolgt zweckmäßigerweise zur Wasserphase vor dem Emulgieren.

### Fleisch- und Fischerzeugnisse

Fleisch- und Fischerzeugnisse, z. B. Pasteten und Fischmarinaden lassen sich in den meisten Fällen mit Mengen bis zu 0,5 bis 10 g/kg der erfindungsgemäßen Salze konservieren. Bei besonders verderbsempfindlichen Produkten wie gekochten Krustentieren können allerdings bis zu 10 g/kg erforderlich sein.

### Oberflächenbehandlung und Überzüge

Die Oberflächenbehandlung von Lebensmitteln, z. B. Fleischerzeugnissen wie gereiften Würsten oder Schinken und Trockenfleisch, kann in der gleichen Art und mit den gleichen Mengen wie bei gereiften Käsen erfolgen. Zur Bildung von Überzügen auf Lebensmitteln wie zum Beschichten von Verpackungsmitteln werden zweckmäßigerweise Trägerstoffe oder Überzugs- oder Filmbildner eingesetzt. Dafür eignen sich insbesondere Stärke, Stärkeether, oxidierte oder abgebaute Stärke, Celluloseether, Alginate, Gellan, Gelatine und Polyvinylalkohol.

### Verpackungen

Durch Einbringen der Calciumdoppelsalze in Überzüge von Lebensmittel oder technischen Produkten sowie Verpackungsmaterialien wird ein fungistatischer Effekt erzielt. Mit etwa 1 bis 10 g Calciumdoppelsalz/m² wird bei Verpackungsfolie oder - papier eine Schutzwirkung gegen Schimmelpilzbildung unter der Verpackung erreicht, wenn diese im direkten Kontakt mit dem verpackten Gut stehen. Die beschichtete Verpackung kann sowohl zur Frischhaltung von frischen Lebensmitteln wie Fleisch, Fisch etc. verwendet werden, als auch für abgepackte Lebensmittel der verschiedensten Art wie Backwaren, Teigwaren und convenience food im weitesten Sinne.

### Tierfutter und als Futtermittel geeignete andere Produkte

Der Zusatz der erfindungsgemäßen Salze verlängert ebenso die Haltbarkeit von Tierfutter. Dazu gehören auch Produkte die zur Verwendung als Futtermittel geeignet sind wie z. B. Silagen, Biertreber, Trester, Bierhefe, Destillationsschlempe und verschiedene Lebensmittelabfälle. Die Salze können in geeigneter Pulverform trocken dem Futter zugemischt werden, vor einer weiteren Verarbeitung (z.B. Extrusion) zugegeben werden oder in Lösung aufgesprüht bzw. im Gemisch gelöst zudosiert werden. Für diese Zwecke werden Konzentrationen bis zu 0,2 bis 50 g/kg, vorzugsweise 0,5 bis 5 g/kg angewendet.

### Kosmetika und Arzneimittel

Zur Konservierung von wasserhaltigen kosmetischen und pharmazeutischen Mitteln (auch Arzneimitteln), werden diesen zur Haltbarkeitsverlängerung die erfindungsgemäßen Calciumdoppelsalze in einer Konzentration von 0,2 bis 50 g/kg, vorzugsweise 0,5 bis 10 g/kg, insbesondere 0,5 bis 5 g/kg, zugesetzt.

### Bedarfsgegenstände und technische Produkte

Zur Konservierung von Bedarfsgegenständen, auch solchen die zur Reinigung und Pflege von Maschinen und Geräten in der Lebensmittelindustrie vorgesehen sind, wie für mikrobiell anfällige technische Erzeugnisse können die erfindungsgemäßen Calciumdoppelsalze in Konzentrationen von bis zu 0,1 bis 50 g/kg, vorzugsweise 0,5 bis 10 g/kg zugesetzt werden.

Die Erfindung wird durch die folgenden Beispiele erläutert:

### Beispiel 1:

### Herstellung eines Calciumdoppelsalzes aus Sorbinsäure und Essigsäure

158 g (1 mol) Calciumacetat werden in 420 ml Wasser gelöst. Zu dieser Lösung wird bei 25 °C eine Lösung aus 56 g (0,37 mol) Kaliumsorbat in 60 ml Wasser zugegeben. Bei der Zugabe bildet sich sofort ein Niederschlag, der nach 5 min abgefiltert wird. Der Niederschlag wird bei 100 mbar und 50°C bis zur Gewichtskonstanz getrocknet. Man erhält 88 %iges Calciumacetatsorbat als weißes Pulver in 73 % Ausbeute.

### Beispiel 2:

### Herstellung eines Calciumdoppelsalzes aus Sorbinsäure und Propionsäure

186 g (1 mol) Calciumpropionat werden in 500 ml Wasser gelöst. Zu dieser Lösung wird bei 25 °C eine Lösung aus 56 g (0,37 mol) Kaliumsorbat in 60 ml Wasser zugegeben. Bei der Zugabe bildet sich ein Niederschlag, der nach 2 min abgefiltert wird. Der Niederschlag wird bei 150 mbar und 50°C bis zur Gewichtskonstanz getrocknet. Man erhält 90 %iges Calciumpropionatsorbat als weißes Pulver in 75 % Ausbeute.

### Beispiel 3:

### Herstellung eines Calciumdoppelsalzes aus Propionsäure und Benzoesäure

186 g (1 mol) Calciumpropionat werden in 500 ml Wasser gelöst. Dazu wird eine Lösung von 57,6 g (0,4 mol) Natriumbenzoat in 70 ml Wasser gegeben. Der sofort entstehende Niederschlag wird nach 2 min abgefiltert und anschließend wie in Beispiel 2 getrocknet. Man erhält 75 g des Doppelsalzes als weißes Pulver.

### Beispiel 4:

### Herstellung eines Calciumdoppelsalzes aus Sorbinsäure und Ameisensäure

130 g (1 mol) Calciumformiat werden in 550 ml Wasser gelöst. Dazu wird eine gesättigte Lösung von 60 g (0,4 mol) Kaliumsorbat gegeben. Der sofort entstehende Niederschlag wird nach 5 min abgefiltert und anschließend wie in Beispiel 1 getrocknet. Man erhält 60 g des Doppelsalzes als weißes Pulver.

### Beispiel 5

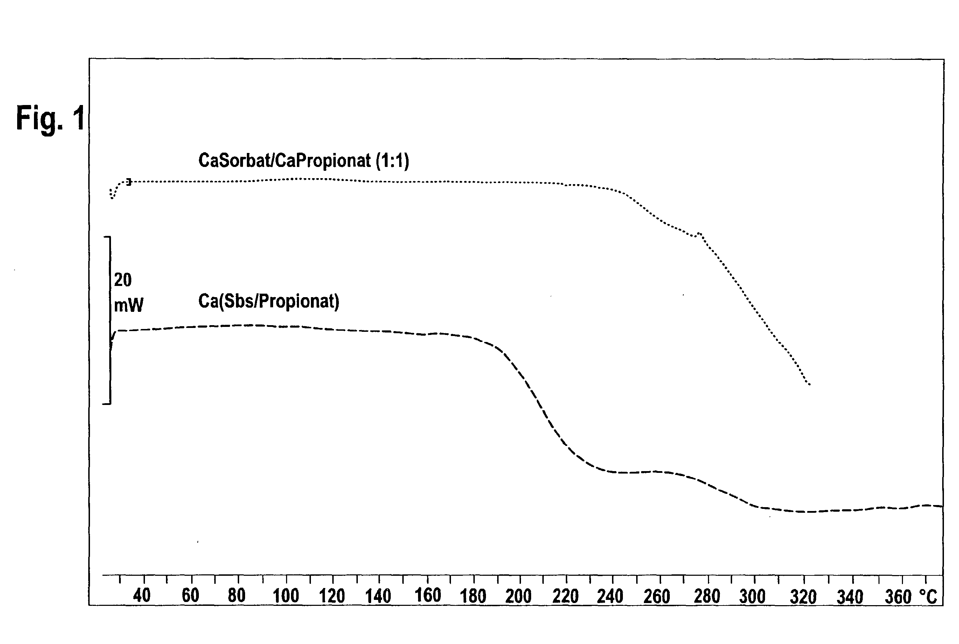

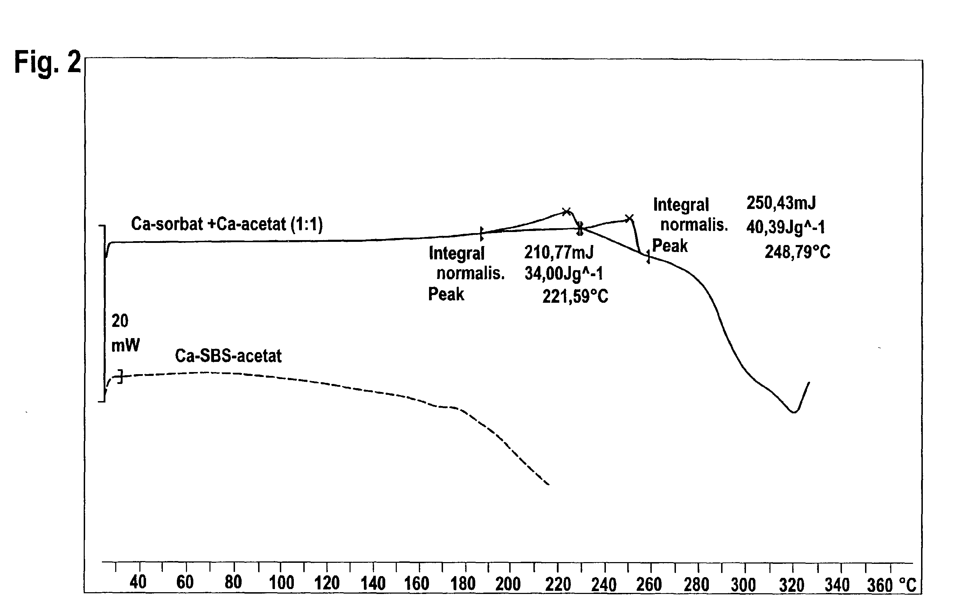

Die hergestellten Doppelsalze wurden mit Mischungen aus den entsprechenden Calciumsalzen mittels Differenzkalorimetrie (DSC) verglichen. Es zeigten sich dabei grundsätzlich geänderte Schmelz- bzw. Zersetzungstemperaturen.
Im Anhang sind die Bilder der DSC von
1) dem Calciumdoppelsalz aus Sorbinsäure und Propionsäure (Figur 1) und
2) dem Calciumdoppelsalz aus Sorbinsäure und Essigsäure jeweils im Vergleich zu den 1:1 Mischungen der Calciumsalze dargestellt (Figur 2).

### Beispiel 6:

### Lagerstabilität der Calciumdoppelsalze

Nach Beispiel 1 hergestellte Salze wurden getrocknet, ohne Zusatz anderer Stoffe unter den angegebenen Bedingungen gelagert und in regelmäßigen Abständen visuell auf Verfärbungen geprüft:

| | | |
|---|---|---|
| Art des Salzes | Zeit nach Gelbbraunverfärbung des Pulvers, verschlossen, - Dunkel bei 25 °C/7 °C - Unter Sonnenlicht bei 25 °C/7 °C | Zeit nach Gelbbraunverfärbung einer1%-igen wäßrigen Lösung, verschlossen, - Dunkel bei 25 °C/7 °C - Unter Sonnenlicht bei 25 °C/7 °C |
| Calciumdoppelsalz aus Sorbin- und Essigsäure | 12 Monate/> 12 Monate 12 Monate / > 12 Monate | >12 Monate / > 12 Monate 12 Monate / > 12 Monate |
| entsprechende "getränkten Salze" nach DE 197 39 319. | 2 Monate / 2 Monate 4-5 Wochen / 2 Monate | 3-4 Wochen / - 3-4 Wochen / - |
| Calciumdoppelsalz aus Benzoe- und Propionsäure. | 7 Monate / 12 Monate 8 Monate / 12 Monate | 8 Monate / 12 Monate 7-8 Monate / 12 Monate |
| entsprechende "getränkten Salze" nach DE 197 39 319 | 5 Wochen / 7 Wochen 6 Wochen / 7 Wochen | 6 Wochen / 8 Wochen 7 Wochen / 7 Wochen |
| Calciumdoppelsalz aus Sorbin- und Propionsäure | 12 Monate / >12 Monate 12 Monate / 12 Monate | >12 Monate / >12 Monate 12 Monate / 12 Monate |
| entsprechende "getränkten Salze" nach DE 197 39 319 | 1-2 Wochen / 4-5 Wochen 1-2 Wochen / 3-4 Wochen | 2-3 Wochen / 4-5 Wochen 3-4 Wochen / 4-5 Wochen |
| Calciumdoppelsalz aus Ameisen- und Sorbinsäure | > 12 Monate / > 12 Monate 12 Monate / > 12 Monate | 12 Monate / > 12 Monate 12 Monate / > 12 Monate |
| entsprechende "getränkten Salze" nach DE 197 39 319 | 4-5 Wochen / 2 Monate 4-5 Wochen / 2 Monate | 3-4 Wochen / 3-4 Wochen 4-5 Wochen / - |

Die in DE 19 739 319 beschriebenen getränkten Salze waren ohne Zusatz von Stabilisatoren in allen Fällen unter diesen Bedingungen i.a. spätestens nach 2 Monaten deutlich verfärbt.

### Beispiel 7:

### Prüfung auf konservierende Wirkung

### Teil I:

Die konservierende Wirkung der Doppelsalze (hier: Teil I, Vergleich zwischen Calciumdoppelsalz aus Sorbinsäure und Propionsäure mit Kaliumsorbat) wurde in einem Modellebensmittel geprüft. Dafür wurde ein auf pH 4,5 eingestellter Apfelsaft verwendet. Von den Prüfkeimen wurden Keimsuspensionen in NaCI-Pepton-Puffer mit einem Zielgehalt von 10^{^{.}6}. KBE/ ml Keimsuspension hergestellt. Je 50 ml Probe bzw. Kontrolle wurden mit 0,5 ml der einzelnen Keimgemische beimpft, homogenisiert und bei 25 °C inkubiert. Für die Belastung wurden folgende Keimsuspensionen zu gleichen Teilen zusammengegeben:

| Keimgemisch | | KBE/ml |
|---|---|---|
| | Lactobacillus acidophilus DSM 20079 | 1,3 x 10⁶ |
| | Lactobacillus delbrückii sp. bulgaricum DSM 20081 | 2,2 x 10⁶ |
| | Leuconostoc mesenteroides DSM 20343 | 2,3 x 10⁶ |
| Summe Milchsäurebakterien | | 1,9 x 10⁶ |
| | Clostridium perfringens DSM 63442 | 1,1 x 10⁶ |
| | Clostridium sporogenes ATCC 10543 | 1,4 x 10⁶ |
| Summe Clostridien | | 1,2 x 10⁶ |
| | Bacillus cereus DSM 345 | 1,2 x 10⁶ |
| | Bacillus subtilis DSM 374 | 1,8 x 10⁶ |
| Summe Bacillen | | 1,5 x 10⁶ |
| Summe thermophile Bacillen | Bacillus stearothermophilus DSM 5934 | 1,3 x 10⁶ |
| | Acetobacter pasteurianus DSM 3509 | 2,7 x 10⁶ |
| | Acetobacter aceti DSM 3508 | 2,1 x 10⁶ |
| | Gluconobacter oxidans DSM 3504 | 2,1 x 10⁶ |
| Summe Essigsäurebakterien: | | 2,3 x 10⁶ |
| | Saccharomyces cerevisiae DSM 70451 | 2,8 x 10⁶ |
| | Zygosaccharomyces rouxii DSM 7525 | 1,9 x 10⁶ |
| | Candida albicans ATCC 10231 | 3,6 x 10⁶ |
| | Brettanomyces naardensis CBS 733 | 2,3 x 10⁶ |
| | Pichia anomala CBS 1683 | 2,0 x 10⁶ |
| Summe Hefen | | 2,5 x 10⁶ |
| | Aspergillus niger ATCC 16404 | 1,2 x 10⁶ |
| | Cladosporium herbarum ATCC 63442 | 1,8 x 10⁶ |
| | Fusarium oxysporum | 1,6 x 10⁶ |
| | Penicillium chrysogenum DSM 895 | 2,1 x 10⁶ |
| Summe Schimmelpilze Keimgemisch I | | 1,7 x 10⁶ |
| | Talaromyces flavus CBS 31763 | 1,9 x 10⁶ |
| | Rhizopus stolonifer DSM 2194 | 1,2 x 10⁶ |
| | Mucor racemosus CBS 11508 | 1,3 x 10⁶ |
| Summe Schimmelpilze Keimgemisch II | | 1,5 x 10⁶ |
| | Escherichia coli ATCC 8739 | 2,4 x 10⁶ |
| | Klebsiella pneumoniae ATCC 10031 | 1,8 x 10⁶ |
| | Citrobacter diversus | 2,6 x 10⁶ |
| | Enterobacter aerogenes DSM 30053 | 2,8 x 10⁶ |
| Summe gram-negatives Keimgemisch: | | 2,4 x 10⁶ |
| Summe Campylobacter | Campylobacter jejuni DSM 4688 | 1,9 x 10⁶ |
| | Staphylococcus aureus ATCC 6538 | 2,4 x 10⁶ |
| | Enterococcus faecalis DSM 20478 | 2,9 x 10⁶ |
| | Streptococcus mutans DSM 50523 | 2,2 x 10⁶ |
| | Listeria monocytogenes ATCC 7644 | 2,8 x 10⁶ |
| Summe gram-positives Keimgemisch: | | 2,6 x 10⁶ |

Zur Bestimmung der Anzahl überlebender Prüfkeime wurde aus den Ansätzen mit dem entsprechenden Keimgemisch jeweils 1 ml Probenmaterial entnommen und mit 9 ml Pepton-Wasser + N9 (4 % Tween 80, 0,5 % Soja-Lecithin) vermischt. Diese Prüflösungen bzw. Verdünnungen aus den Prüflösungen wurden auf den entsprechenden Agar ausgespatelt oder als Gussplatte mit dem entsprechenden Nähragar vermischt. Die Bebrütungsdauer und Bebrütungstemperaturen sind in der folgenden Tabelle aufgelistet:

| Mikroorganismen | Agar | Bebrütungsdauer | Bebrütungstemperatur |
|---|---|---|---|
| Milchsäurebakterien | Caso-HLT | 3 Tage | 32 °C |
| Clostridien | DRCM | 3 Tage | 32 °C |
| Bacillen | Caso-HLT | 3 Tage | 32 °C |
| Thermophile Bacillen | Caso-HLT | 3 Tage | 55 °C |
| Essigsäurebakterien | YPM-Agar | 3 Tage | 32 °C |
| Hefen | Sabouraud-HLT | 3-5 Tage | 25 °C |
| Schimmelpilze | Sabouraud-HLT | 3-5 Tage | 25 °C |
| Gram-negative Keime | Caso-HLT | 3 Tage | 32 °C |
| Campylobacter | Anaerocult C auf CCDA | 3 Tage | 42 °C |
| Gram-positive Keime | Caso-HLT | 3 Tage | 32 °C |

HLT = 3 % Tween 80, 0,5 % Lecithin, 0,1 % Histidin

Die Anzahl der überlebenden Mikroorganismen wurde 1 Tag (24 h), 3 Tage, 1 und 2 Wochen nach der Inokulation ermittelt. Dabei wurden die folgenden Ergebnisse erhalten:

### Apfelsaft ohne Zusätze

Gehalt überlebender Keime pro Gramm Probe (KBE/g) nach Kontamination

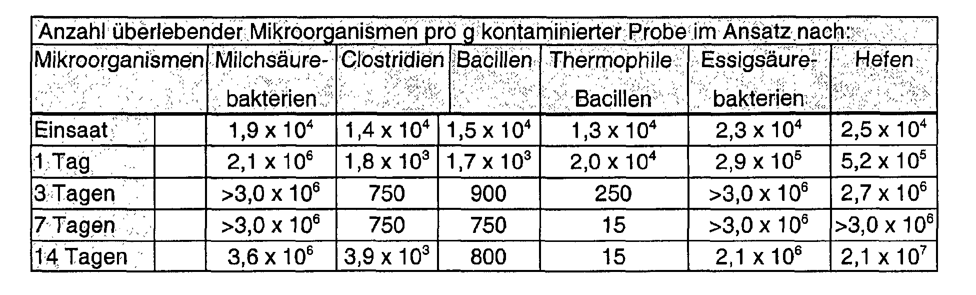

Gehalt überlebender Keime pro Gramm Probe (KBE/g) nach Kontamination

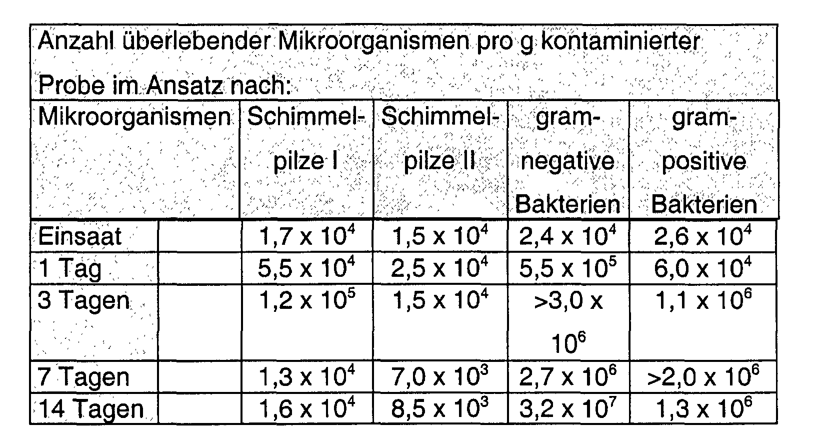

### Zusatz von 1 g/l Calciumdoppelsalz aus Sorbinsäure und Propionsäure

Gehalt überlebender Keime pro Gramm Probe (KBE/g) nach Kontamination

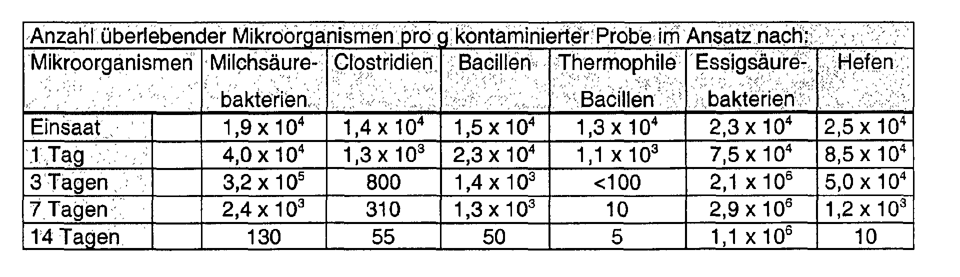

Gehalt überlebender Keime pro Gramm Probe (KBE/g) nach Kontamination

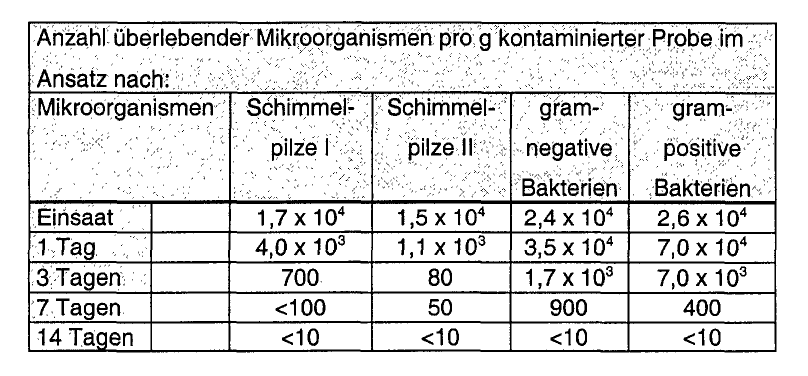

### Zusatz von 1 g/l Kaliumsorbat

Gehalt überlebender Keime pro Gramm Probe (KBE/g) nach Kontamination

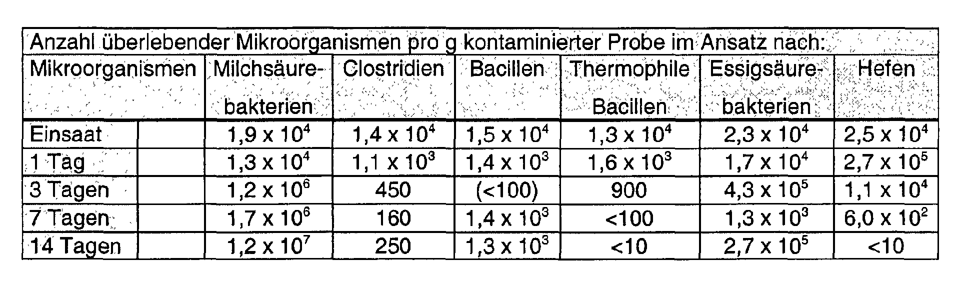

Gehalt überlebender Keime pro Gramm Probe (KBE/g) nach Kontamination

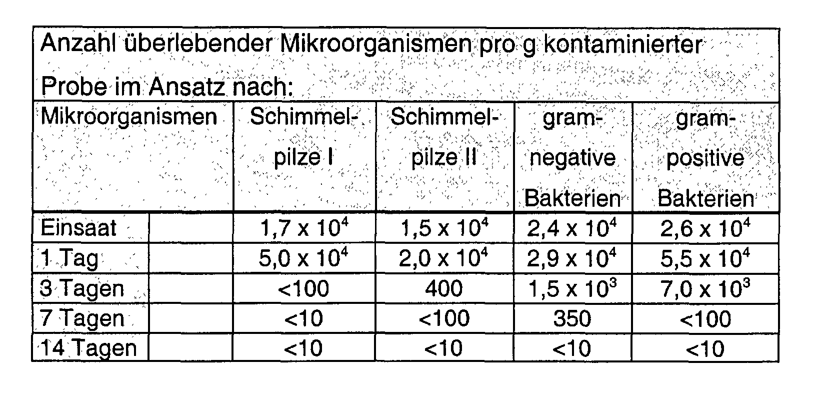

Die Ergebnisse dieser Prüfungen zeigen, dass das Calciumdoppelsalz aus Propionsäure und Sorbinsäure gegen alle eingesetzten Mikroorganismen, ausgenommen Bacillen gut und nachhaltig wirkt. Im Falle einer Kontamination wirkt dieses Doppelsalz überraschender Weise besser als Kaliumsorbat (eingesetzt in gleicher Konzentration), insbesondere gegen Milchsäurebakterien, thermophile Bazillen, Hefen und einige Schimmelpilze (Schimmelpilze Gruppe II).

### Prüfung auf konservierende Wirkung

### Teil II:

In einem zweiten Versuch wurde die konservierende Wirkung der Doppelsalze (hier: Vergleich zwischen Calciumdoppelsalz aus Sorbinsäure und Essigsäure oder Ameisensäure mit den entsprechenden Calciumsalzen) in dem oben beschriebenen Modelllebensmittel geprüft.

Für die Belastung wurden folgende Keimsuspensionen zu gleichen Teilen zusammengegeben:

| Keimgemisch | | KBE/ml |
|---|---|---|
| | Lactobacillus acidophilus DSM 20079 | 2,7 x 10⁶ |
| | Lactobacillus delbrückii sp bulgaricum DSM 20081 | 2,5 x 10⁶ |
| | Leuconostoc mesenteroides DSM 20343 | 2,9 x 10⁶ |
| Summe Milchsäurebakterien: | | 2,7 x 10⁶ |
| | Clostridium perfringens DSM 63442 | 2,9 x 10⁶ |
| | Clostridium sporogenes ATCC 10543 | 2,9 x 10⁶ |
| Summe Clostridien: | | 2,9 x 10⁶ |
| | Bacillus cereus DSM 345 | 3,4 x 10⁶ |
| | Bacillus subtilis DSM 374 | 3,2 x 10⁶ |
| Summe Bacillen: | | 3,3 x 10⁶ |

| Keimgemisch | | KBE/ml |
|---|---|---|
| Summe thermophile Bacillen: | Bacillus stearothermophilus DSM 5934 | 1,3 x 10⁶ |
| | Acetobacter pasteurianus DSM 3509 | 2,4 x 10⁶ |
| | Acetobacter aceti DSM 3508 | 2,3 x 10⁶ |
| | Gluconobacter oxidans DSM 3504 | 3,8 x 10⁶ |
| Summe Essigsäurebakterien: | | 2,8 x 10⁶ |
| | Saccharomyces cerevisiae DSM 70451 | 2,6 x 10⁶ |
| | Zygosaccharomyces rouxii DSM 7525 | 2,9 x 10⁶ |
| | Candida albicans ATCC 10231 | 3,1 x 10⁶ |
| | Brettanomyces naardensis CBS 733 | 2,5 x 10⁶ |
| | Pichia anomala CBS 1683 | 1,9 x 10⁶ |
| Summe Hefen: | | 2,6 x 10⁶ |
| | Aspergillus niger ATCC 16404 | 2,9 x 10⁶ |
| | Cladosporium herbarum ATCC 63442 | 1,6 x 10⁶ |
| | Fusarium oxysporum | 2,1 x 10⁶ |
| | Penicillium chrysogenum DSM 895 | 2,2 x 10⁶ |
| Summe Schimmelpilze Keimgemisch I: | | 2,2 x 10⁶ |
| | Talaromyces flavus CBS 31763 | 2,7 x 10⁶ |
| | Rhizopus stolonifer DSM 2194 | 2,4 x 10⁶ |
| | Mucor racemosus CBS 11508 | 2,4 x 10⁶ |
| Summe Schimmelpilze Keimgemisch II: | | 2,5 x 10⁶ |
| | Escherichia coli ATCC 8739 | 2,1 x 10⁶ |
| | Klebsiella pneumoniae ATCC 10031 | 2,7 x 10⁶ |
| | Citrobacter diversus | 3,4 x 10⁶ |
| | Enterobacter aerogenes DSM 30053 | 2,7 x 10⁶ |
| Summe gram-negatives Keimgemisch: | | 2,7 x 10⁶ |
| | Staphylococcus aureus ATCC 6538 | 2,4 x 10⁶ |
| | Enterococcus faecalis DSM 20478 | 1,8 x 10⁶ |
| | Streptococcus mutans DSM 50523 | 1,9 x 10⁶ |
| | Listeria monocytogenes ATCC 7644 | 2,3 x 10⁶ |
| Summe gram-positives Keimgemisch: | | 2,1 x 10⁶ |

Des weiteren wurde die Untersuchung wie oben im Teil I beschrieben durchgeführt. Die Anzahl der überlebenden Mikroorganismen wurde 1 Tag (24 h), 3 Tage, 1 und 2 Wochen nach der Inokulation ermittelt. Dabei wurden die folgenden Ergebnisse erhalten:

### Apfelsaft ohne Zusätze

Gehalt überlebender Keime pro Gramm Probe (KBE/g) nach Kontamination

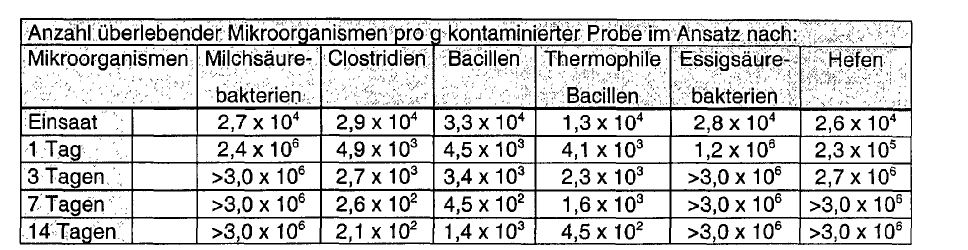

Gehalt überlebender Keime pro Gramm Probe (KBE/g) nach Kontamination

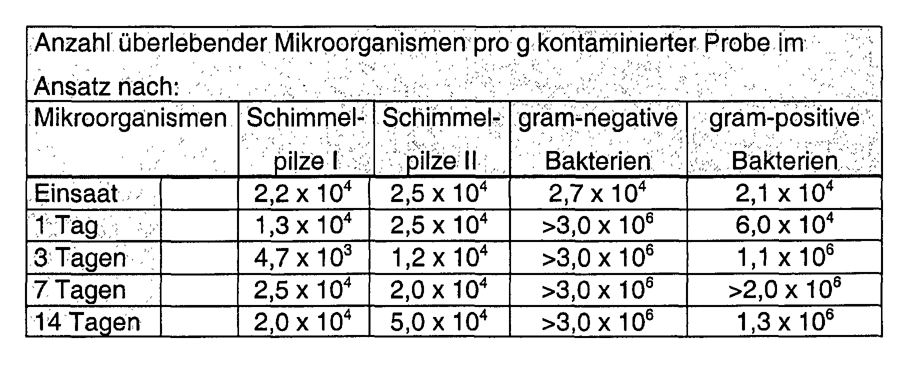

### Zusatz von 1 g/l Calciumacetat

Gehalt überlebender Keime pro Gramm Probe (KBE/g) nach Kontamination

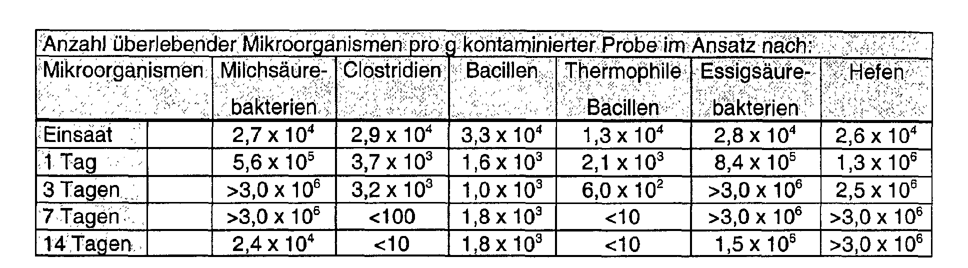

Gehalt überlebender Keime pro Gramm Probe (KBE/g) nach Kontamination

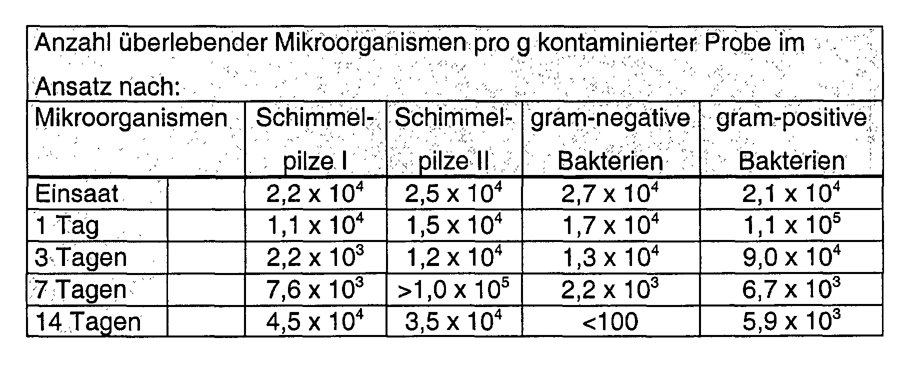

### Zusatz von 1 g/l Calciumsorbat

Gehalt überlebender Keime pro Gramm Probe (KBE/g) nach Kontamination

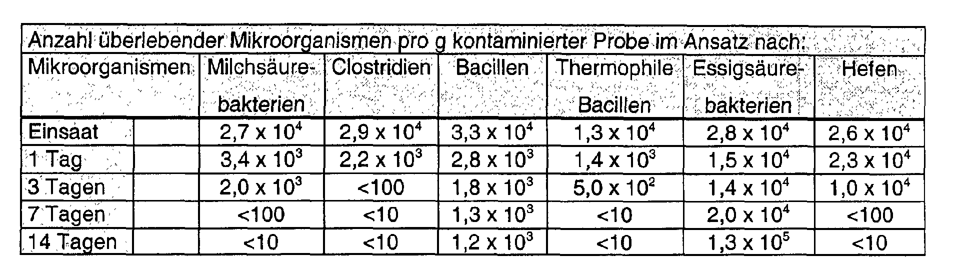

Gehalt überlebender Keime pro Gramm Probe (KBE/g) nach Kontamination

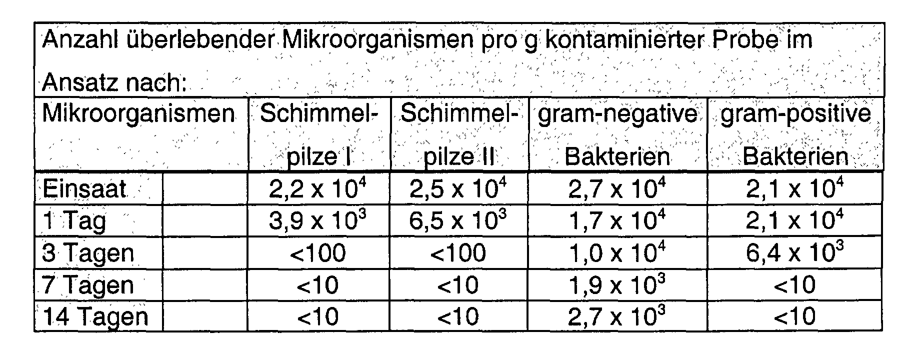

### Zusatz von 1 g/l Calciumdoppelsalz aus Sorbinsäure und Essigsäure

Gehalt überlebender Keime pro Gramm Probe (KBE/g) nach Kontamination

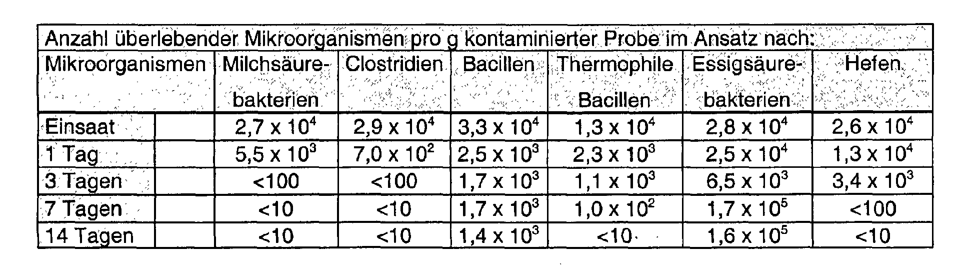

Gehalt überlebender Keime pro Gramm Probe (KBE/g) nach Kontamination

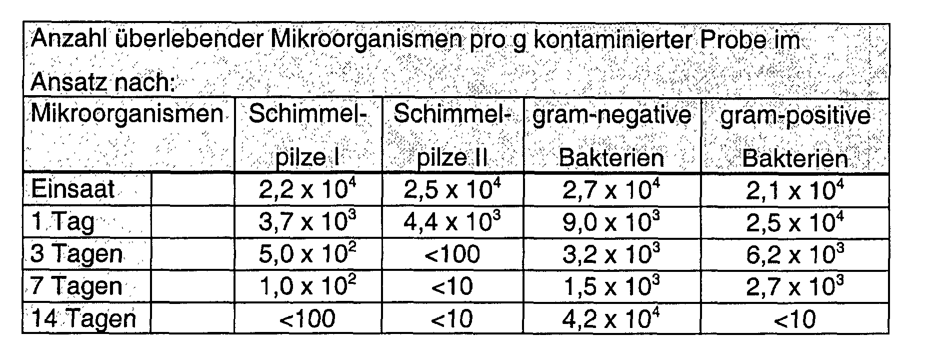

Die Ergebnisse dieser Prüfungen zeigen, dass das Calciumdoppelsalz aus Essigsäure und Sorbinsäure gegen alle eingesetzten Mikroorganismen, ausgenommen Essigsäurebakterien und Bacillen gut und nachhaltig wirkt. Das Wachstum gramnegativer Bakterien wird weitgehend verhindert. Im Falle einer Kontamination wirkt dieses Doppelsalz auch überraschenderweise besser als Calciumacetat (insbesondere gegen Milchsäurebakterien, Hefen, Schimmelpilze und grampositive Bakterien) bzw. Calciumsorbat (insbesondere gegen Milchsäurebakterien) alleine eingesetzt.

### Zusatz von 1 g/l Calciumformiat

Gehalt überlebender Keime pro Gramm Probe (KBE/g) nach Kontamination

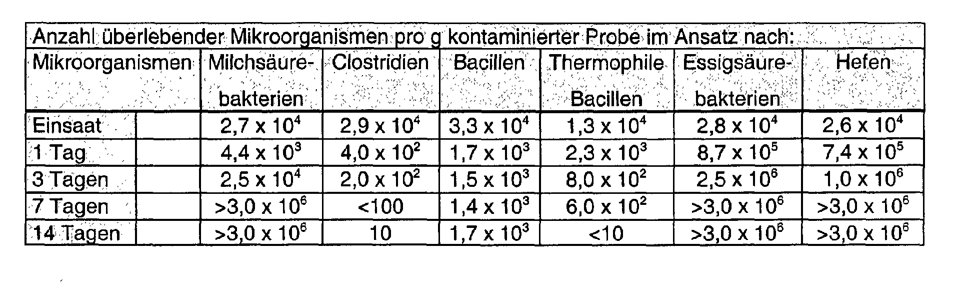

Gehalt überlebender Keime pro Gramm Probe (KBE/g) nach Kontamination

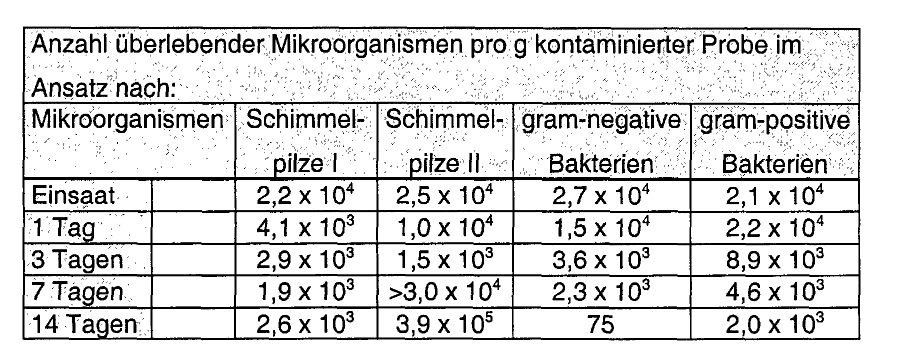

### Zusatz von 1 g/l Calciumdoppelsalz aus Sorbinsäure und Ameisensäure

Gehalt überlebender Keime pro Gramm Probe (KBE/g) nach Kontamination

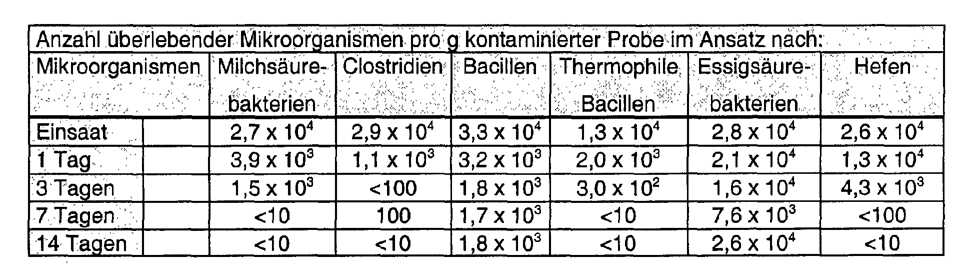

Gehalt überlebender Keime pro Gramm Probe (KBE/g) nach Kontamination

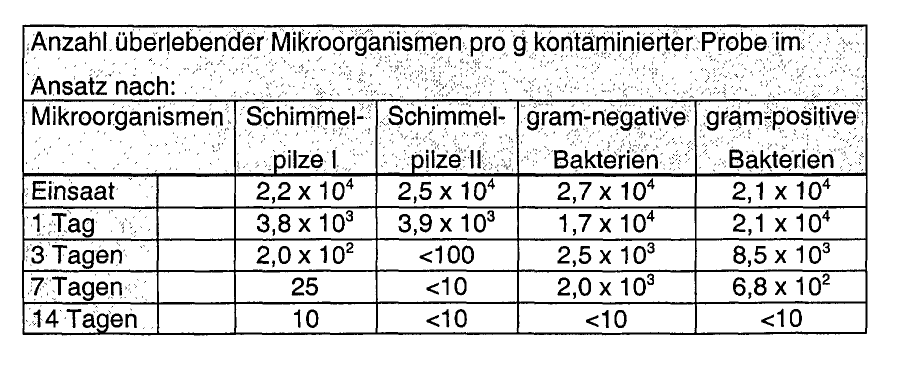

Die Ergebnisse dieser Prüfungen zeigen, dass das Calciumdoppelsalz aus Ameisensäure und Sorbinsäure gegen alle eingesetzten Mikroorganismen, ausgenommen Essigsäurebakterien und Bacillen gut und nachhaltig wirkt. Im Falle einer Kontamination wirkt dieses Doppelsalz auch überraschenderweise besser als Calciumformiat (insbesondere gegen Milchsäurebakterien, Hefen und Schimmelpilze) bzw. Calciumsorbat (insbesondere gegen gramnegative Bakterien) alleine eingesetzt.

## Patentansprüche

1. Calciumdoppelsalze organischer (Konservierungsstoff)säuren der Formel I
Ca(R¹)(R²) (I)
worin R¹ und R² verschieden sind und jeweils OOC-R³ bedeuten, mit R³ = gesättigtes oder ein- oder mehrfach ungesättigtes C₁-C₅-Alkyl, C₁-C₅-Hydroxyalkyl oder Phenyl.

2. Salze nach Anspruch 1 , **dadurch gekennzeichnet, dass** R³ Methyl, Ethyl, Hydroxyethyl, Propyl, 1,3-Hexadienyl (=Sorbatanion) oder Phenyl bedeutet.

3. Salze nach Anspruch 1 oder 2, **dadurch gekennzeichnet, dass** R¹ Propionat und R² Sorbat bedeutet.

4. Verfahren zur Herstellung der Salze gemäß Anspruch 1, **dadurch gekennzeichnet, dass** eine Lösung des in Wasser besser löslichen Calciumsalzes mit einer Lösung des Alkalisalzes der zweiten Carbonsäure miteinander gemischt werden und der entstehende Niederschlag abgetrennt und getrocknet wird.

5. Verfahren nach Anspruch 4, **dadurch gekennzeichnet, dass** die eingesetzten Lösungen möglichst gesättigt sind.

6. Verwendung der Salze gemäß Anspruch 1 zur Konservierung von Lebensmitteln, Arzneimitteln, Futtermitteln, Silagen, Biertrebern, Trester, Lebensmittelabfällen, Bierhefe, Destillationsschlempen, Kosmetika, Leder und technischen Produkten.

7. Lebensmittel, **dadurch gekennzeichnet, dass** es ein Salz gemäß Anspruch 1 enthält.

8. Arzneimittel, **dadurch gekennzeichnet, dass** es ein Salz gemäß Anspruch 1 enthält.

9. Futtermittel, **dadurch gekennzeichnet, dass** es ein Salz gemäß Anspruch 1 enthält.

10. Silage, **dadurch gekennzeichnet, dass** sie ein Salz gemäß Anspruch 1 enthält.

11. Biertreber, **dadurch gekennzeichnet, dass** er ein Salz gemäß Anspruch 1 enthält.

12. Trester, **dadurch gekennzeichnet, dass** er ein Salz gemäß Anspruch 1 enthält.

13. Lebensmittelabfall, **dadurch gekennzeichnet, dass** er ein Salz gemäß Anspruch 1 enthält.

14. Bierhefe, **dadurch gekennzeichnet, dass** sie ein Salz gemäß Anspruch 1 enthält.

15. Destillationsschlempe, **dadurch gekennzeichnet, dass** sie ein Salz gemäß Anspruch 1 enthält.

16. Kosmetikum, **dadurch gekennzeichnet, dass** es ein Salz gemäß Anspruch 1 enthält.

17. Fettemulsion, **dadurch gekennzeichnet, dass** es ein Salz gemäß Anspruch 1 enthält.

18. Technisches Produkt (Folie, fungistatischer Überzug), **dadurch gekennzeichnet, dass** es ein Salz gemäß Anspruch 1 enthält.

19. Zubereitung, enthaltend eine oder mehrere der folgenden Substanzen: Stärke, Stärkederivat, Celluloseether, Polysaccharid, Polyvinylalkohol sowie ein Salz gemäß Anspruch 1.
